# EUROPEAN PATENT SPECIFICATION

(11) **EP 0 878 208 B1**
(45) Date of publication and mention of the grant of the patent: **22.12.2004**
(21) Application number: 98303848.0
(22) Date of filing: 15.05.1998
(51) Int. Cl.: A61M 16/12, A61M 16/00

(54) **Constant volume nitric oxide pulse delivery device**
Vorrichtung zur Verabreichung von Stickoxidpuls mit konstantem Volumen
Dispositif de distribution de pulse de volume constant d'oxide nitrique

(30) Priority: 16.05.1997 US 857924
(43) Date of publication of application: 18.11.1998
(73) Proprietor: Datex-Ohmeda, Inc., Tewsbury, Massachusetts 01876 (US)
(72) Inventor: Bathe, Duncan P.L., Wisconsin 53711 (US); Montgomery, Frederick J., Sun Prairie, Wisconsin 53590 (US)
(74) Representative: Hedley, Nicholas James Matthew

(56) References cited:
- EP-A- 0 602 734
- WO-A-95/28193
- GB-A- 2 240 849
- US-A- 4 823 788
- US-A- 5 603 315

## Description

### BACKGROUND

This invention relates to the administration of a therapeutic gas such as nitric oxide (NO) to patients for therapeutic effect. In particular, it relates to a system provided with an alarm to indicate faults. Preferably a controlled, predetermined supplemental volume of a dose of NO is provided to the patient with each inhalation by the patient.

The function of the administration of NO has been fairly widely published and typical articles appeared in The Lancet, Vol. 340, October 1992 at pages 818-820 entitled "Inhaled Nitric Oxide in Persistent Pulmonary Hypertension of the Newborn" and "Low-dose Inhalational Nitric Oxide in Persistent Pulmonary Hypertension of the. Newborn" and in Anesthesiology, Vol. 78, pgs. 413-416 (1993), entitled "Inhaled NO-the past, the present and the future".

The actual administration of NO is generally carried out by its introduction into the patient as a gas and commercially available supplies are provided in cylinders under pressure and may be at pressures of about 2000 psi and consist of a predetermined mixture of NO in a carrier gas such as nitrogen. A pressure regulator is therefore used to reduce the pressure of the supply cylinder to working levels for introduction to a patient.

The concentration administered to a patient will vary according to the patient and the need for the therapy but will generally include concentrations at or lower than 150 ppm. There is, of course, a need for that concentration to be precisely metered to the patient since an excess of NO can be harmful to the patient.

One current known method and apparatus for the administration of NO to patients is described in U.S. Patent 5,558,083 where a system is provided that can be added to any ventilator and which will meter in the desired concentration of NO into the gas supplied from that ventilator.

Various other delivery devices have also been used that respond to the patient attempting to inhale to deliver a pulsed dose of NO to the patient and such pulsing devices have also been shown to have therapeutic effect on the patient, for example, as described in Higenbottam PCT patent application WO 95/10315 and the publication of Channick et al "Pulsed delivery of inhaled nitric oxide to patients with primary pulmonary hypertension", Chest/109/ June 1996. In such pulsatile dosing devices, a pulse of NO is administered to the patient as the patient inhales spontaneously.

The inhalation pulsing type devices are typically shown and described in Durkan, U.S. Patent 4,462,398, however the Durkan device has certain limitations and was designed for the administration of oxygen and not NO. Again, further devices are known, based somewhat on the Durkan device, such as described in Perkins U.S. Patent 5,005,570 and Dietz U.S. Patent 5,038,771. Again, however, the devices were principally designed for use in the administration of oxygen. One of the properties of NO is that it has a toxic effect at high concentrations while having its beneficial therapeutic effect at low concentrations. Nitric oxide systems, therefore, require additional safeguards and control than with devices designed for the delivery of oxygen. In addition, of course, NO reacts with oxygen to form a more toxic compound, NO₂ and again, therefore, greater safety and control requirements are needed than with an oxygen delivery device.

Basically, of the types known, the Durkan and Perkins approach to administration is based on the time that a flow control valve is open and thus the time that the gas is administered to the patient. With such a system, however, the device does not provide a fixed volume of gas to the patient under all conditions as a variation in input pressure or altitude will result in a higher or lower flow of gas through the valve. Accordingly, with a fixed time interval, the differing flow will result in differing volumes being delivered per breath to the patient.

As an example, with a timed interval device the cylinder of gas will generally be reduced in pressure as the administration of the gas is carried out. The regulator that reduces the cylinder pressure to a working pressure, generally puts out an increased pressure as the cylinder pressure is lowered, that is, an inverse function. Since that pressure downstream of the regulator varies, so does the flow to the patient and the actual volume dose of NO to the patient is, therefore, not constant with a constant timed dosage, but varies as the cylinder pressure varies.

The Dietz patent takes a differing approach, and which varies the time the valve is opened based on the size of the previous breath of the patient. Again, the volume delivered per breath is variable due to the input pressure and the altitude and is further dependent on the size of the breath itself, i.e. the larger the breath, the higher the dose provided to the patient. Such may not be required for NO therapy to the patient.

One further problem with the typical prior art devices is that there is no safety system to indicate when the timing valve has failed and thus, the patient may not be receiving the needed therapy from the device or may be receiving an excess of therapy.

Accordingly, with the use of such current devices for administering NO, the actual volume of the NO to the patient may vary and the exact volume is basically unknown, thus the control of the therapy is somewhat difficult and a need exists for more precise control of the volume of NO administered per breath.

US-A-5 603 315 discloses an oxygen delivery system for delivering pulses of oxygen to a conduit containing a valve controlling the pulses of oxygen to a patient in accordance with signals from a micro-controller. The system also contains an alarm that is activated in the case of malfunction.

### SUMMARY OF THE INVENTION

In accordance with the present invention, there is provided a respiratory therapy apparatus for delivering a therapeutic gas, eg nitric oxide, as set out in the accompanying claims. The apparatus includes an alarm indicating faults. Preferably, a constant volume of NO is administered to the patient regardless of changes in the cylinder pressure or other factors affecting the delivery. The NO can be delivered in a cylinder mixed with a carrier gas such a nitrogen without affecting the constant volume administered.

In order to deliver a constant volume of NO, a sensing device detects the start of a patients spontaneous breathing and the system injects a predetermined desired volume of NO to the patient at the start of that breath. That predetermined desired volume may be selected by the user with some input device or may be factory set by the manufacturer of the equipment. In the preferred embodiment, the sensing device may be mounted to the gas cylinder containing NO and a balance gas such as nitrogen. The patient is connected to the outlet of the delivery system by a single connection such as a nasal cannula or similar device for actual administration of the therapeutic gas to the patient.

A central processing unit (CPU) may provide the control of a control valve upon the sensing device indicating the start of an inhalation. The CPU energizes the control valve from the normally closed position where no therapeutic gas is being delivered to the patient to the energized, open position where gas is supplied to the patient for a calculated period of time and at the end of that time period, the CPU switches the control valve from the energized, open position, back to the de-energized, closed position. The flow of NO containing gas flows through a fixed restrictor that is located intermediate the regulator and the patient. The amount of the time during which the control valve is open is calculated by the CPU based on the pressure delivered from the regulator, the ambient pressure, the characteristics of the restrictor and the predetermined desired volume of NO.

In the preferred embodiment, an absolute pressure transducer is used to determine and monitor the pressure upstream of the fixed restrictor. Such transducers operate off a base of 0 absolute pressure and therefore take into account the ambient pressure in the area. Since the flow through a fixed restrictor is directly proportional to the absolute pressure upstream of the regulator, the measurement of that absolute pressure is used by the CPU to precisely measure the flow of NO containing gas to the patient.

The flow through the fixed restrictor is therefore a known value based on the absolute pressure upstream of the restrictor and the known characteristics of the particular restrictor available from the supplier of that restrictor. Accordingly, the measurement of the pressure, along with the known pressure flow characteristics of the restrictor will accurately give the flow of NO containing therapeutic gas delivered to the patient during the time period the control valve is in the open position. That determination of flow can then be integrated with respect to the time the control valve is open by the CPU to calculate the actual volume of NO and carrier gas delivered to the patient.

As can then be seen, given the volume so calculated, that volume can be compared to the predetermined desired volume set by the user or established in the delivery suystem and the control valve open time varied by the CPU to correct for any differences. Thus, the precise volume of NO delivered to the patent can be controlled with respect to each breath of the patient.

At start up, the system CPU may start with the pressure of the previous use of the device or, alternatively, can arbitrarily pick out an absolute pressure that is known to be around the value that is inputted to the fixed restrictor, i.e. around 50 psig. After start-up, the unit will cycle for a few times so that it can calculate the actual values of the volume of gas to the patient and compare that value with the value set by the user and eventually modify the time the control valve is open during each inhalation to have the system provide the exact predetermined delired volume to the patient.

### BRIEF DESCRIPTION OF THE DRAWINGS

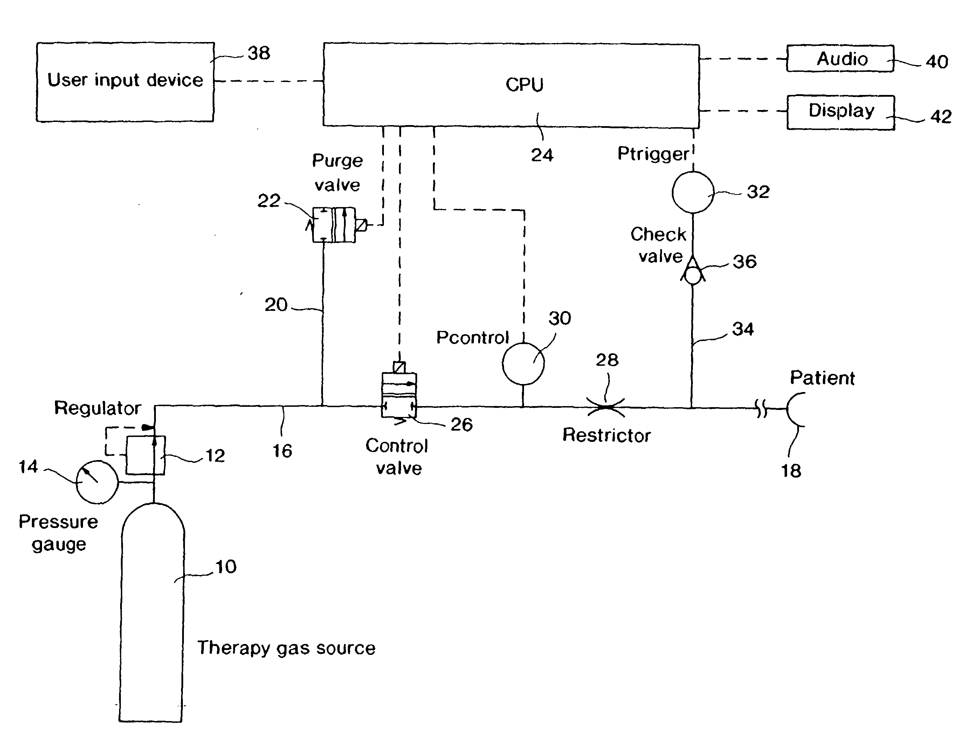

The Figure is a schematic view of a NO delivery system constructed in accordance with the present invention.

### DETAILED DESCRIPTION OF THE INVENTION

Turning now to the Figure, there is shown a schematic view of a pulsed dosing NO delivery apparatus constructed in accordance with the present invention. A gas cylinder 10 is provided containing the therapeutic amount of nitric oxide. Preferable that NO gas is mixed with a balance or carrier gas such as nitrogen and the concentration may be in the order of 100 ppm. The NO in nitrogen gas is available commercially in cylinders at pressures of approximately 2000 psig.

A pressure regulator 12 reduces the cylinder pressure down to a working pressure for use with the present system and that pressure may be in the order of about 50 psig. A pressure gauge 14 is generally provided on the pressure regulator 12 in order to keep track of the pressure within the gas cylinder 10.

A conduit 16 carries the NO containing therapy gas from the regulator 12 through to a patient 18 where the NO containing therapy gas is administered to the patient by means such as a nasal cannula (not shown). Teed off from the conduit 16 is a purge line 20 and a purge valve 22. As can be seen, the purge valve 22 is normally in the non-energized position blocking the flow of gas therethrough and is activated by a CPU 24 to open the purge valve 22 to clear certain portions of the conduit 16 as well as regulator 12 of gas. Thus, when the gas cylinder 10 is opened, on initial use of the equipment, the NO containing therapy gas flows through the regulator 12, a portion of the conduit 16 and, the purge line 20 and is exhausted out the purge valve 22 to rid those passages of air and thus to make sure that the oxygen present in air cannot act on the NO to create NO₂, a toxic substance.

Operation of the purge valve 22 may be immediate by the CPU 24 upon start up of the apparatus or may be accomplished manually with a prompt from a display operated by the CPU 24.

A control valve 26 controls the flow of NO containing therapy gas from the gas cylinder 10 to the patient 18 and is a solenoid controlled valve operated by signal from the CPU 24. Again, for safety, the control valve 26 is normally closed and is moved to its open position when a signal energizes the valve by CPU 24. As will be explained, the time during which the control valve is in the open position controls the volume of NO containing therapy gas to the patient 18.

A fixed restrictor 28 is also provided in the conduit 16 and may be a commercially available restrictor and which is provided with the pressure to flow characteristics by the supplier. Just upstream of the fixed restrictor is an absolute pressure transducer 30 which senses the absolute pressure in the conduit 16 as P_{control.} That pressure is also communicated to the CPU 24 as will be later explained. The absolute pressure transducer 30 is of the type that operates off a base of zero psi and therefore it reads the absolute pressure within the conduit 16 at the point just upstream of the fixed restrictor 28. By the absolute pressure, the reading takes into account the ambient pressure surrounding the apparatus. Typical pressure transducers of the absolute pressure type are available from Sensyn, Inc.

Accordingly, as may now be seen, the CPU 24 is in receipt of all of the information necessary to determine the exact flow of NO containing therapy gas through the fixed restrictor 28 and thus, the flow to the patient 18. The characteristics of the particular fixed restrictor 28, as stated, are available from the manufacturer as a curve or other data that can be inputted to the CPU as a look up table or the like. Since the flow through the fixed restrictor 28 is directly proportional to the absolute pressure of the gas entering the fixed restrictor 28, the CPU also knows the P_{control} from the absolute pressure transducer 30 and thus can readily calculate the flow to the patient 18.

A patient trigger 32 is in communication with the patient by means of a passageway 34 and including a check valve 36. The patient trigger 32 may be of conventional design and basically detects a negative pressure P_{trigger} from the patient indicating that the patient 18 is initiating inhalation. That patient trigger 32 thus provides a signal to the CPU 24 to alert the CPU 24 that the patient is initiating an inhalation so that the CPU can take the appropriate action to provide a pulse of NO containing therapeutic gas to the patient 18 during that inhalation.

A user input device 38 allows the user to input to the CPU 24 the specific volume of NO containing therapeutic gas that is desired to be delivered to the patient 18 during each inhalation and such device may be a rotary switch or the like. Alternatively, the volume to be delivered may be predetermined by the manufacturer of the delivery system and already established in the system so and not be individually selected in the field by a user. Also as a part of the system, there may be an audio alarm 40 and a visual display 42 that may also contain visual alarms as well as display various monitored conditions of the device to the user.

The overall operation of the NO dosing device may now be explained amd will refer to the delivery system embodiment where the user makes the desired selection of the volume to be administered to the patient. As noted, upon start-up of the system, the gas cylinder 10 containing the NO therapy gas in a predetermined concentration is opened and the NO containing therapy gas enters the regulator 12 and the conduit 16. Purge valve 22 is opened by a signal from CPU 24 or manually by a prompt displayed on the visual display 42 so that the pressure regulator and the portion of conduit 16 is purged of air.

The user inputs a volume of NO containing therapy gas that is desired to be administered to the patient 18 by means of the user input device 38. As the patient initiates an inhalation, the patient trigger 32 senses the negative pressure and signals the CPU to commence the injection of a dosage of NO containing therapy gas to the patient 18. Initially, the CPU opens the control valve 26 for a predetermined time based upon a preselected P_{control} such as 65 psia or may use a pressure last used by the CPU 24 and retained in its memory. That P_{control} will cause the CPU 24 to open the control valve 26 for a calculated period of time to allow the administration of the NO containing therapy gas to the patient 18 and then will move the control valve 26 to it closed position.

The CPU 24 can now calculate the exact volume of gas delivered to the patient 18, using the data that is representative of the characteristics of the fixed restrictor 28 and the input it receives from the absolute pressure transducer 30 of P_{control}. With that data and the amount of time that the control valve 26 has been opened, the CPU 24 can readily calculate the exact volume by integrating the flow through fixed restrictor 28 with respect to the time the control valve 26 is in its open position and arrive at the volume of NO containing therapeutic gas administered to the patient 18.

With the calculated volume, the CPU 24 can then compare the volume calculated with the volume that has been inputted by the user as the desired volume for administration to the patient 18. The CPU 24 can thus alter the time the control valve 26 is opened and recalculate until the volume that it calculates is the same as the volume inputted by the user in the user input device 38. At this point, the overall device can administer a user set precise volume of NO containing therapeutic gas at each inhalation triggered by the patient 18.

As further safety features of the NO dosing device, it is possible to detect the failure of the control valve 26 based on data from the P_{control} and the known timing of the control valve sequence. For example, if there is no rise in the value of P_{control} by a known amount, based upon a minimum supply pressure, and the control valve 26 has been given a signal to open by the CPU 24, then the system will recognize a failure to deliver the therapy to the patient 18 and a suitable alarm may be activated at audio alarm 40 and/or by a visual alarm indication on the visual display 42. Conversely, if the P_{control} does not reduce by a known amount, again based upon the minimum supply pressure, and the control valve 26 has been given the signal to close by CPU 24, then the system can detect an over delivery failure and again an audible or visual alarm activated.

Accordingly, the foregoing description of the preferred embodiment should be taken by way of illustration rather than by way of limitation of the invention as claimed.

## Claims

1. A nitric oxide respiratory therapy apparatus for delivering a pulse of nitric oxide therapeutic gas to a patient, said system comprising:
a conduit (16) adapted to be connected to a source of the therapeutic gas (10) under pressure and connected to a patient (18),
said conduit (16) including a valve (26) controlling the flow of the nitric oxide therapeutic gas from a source to a patient,
control means (24) to open and close said valve (26) at timed intervals by sending a signal to open said valve and a signal to close said valve,
a pressure sensor (30) adapted to detect the pressure within said conduit (16) downstream of said control valve (26),
an alarm (40, 42)
**characterised in that** the apparatus further includes:
means to activate said alarm indicating a failure of the valve (26) when (a) said signal has been sent by said control means (24) to close said valve (26) and said pressure sensor (30) does not detect a drop in pressure downstream of said control valve by a predetermined amount indicative that the valve (26) has not closed and/or (b) said signal has been sent by said control means (24) to open said valve (26) and s aid p ressure s ensor d oes not d etect a n increase i n pressure downstream of said control valve by a predetermined amount indicative that the valve (26) has not opened.

2. A respiratory therapy apparatus as claimed in claim 1, wherein the control means includes timing means (24) to control the duration of the time the delivery means is providing the therapeutic gas to a patient, and wherein the apparatus further includes:
means (38) to establish a predetermined desired volume of a therapeutic gas to be delivered to a patient during each inhalation,
sensing means (32) for sensing the initiation of an inhalation of a patient, the control means (24) and the valve (26) being responsive to said sensing means (32) sensing an inhalation of a patient to deliver a volume of the therapeutic gas to a patient during each inhalation of a patient,
volume monitoring means (24) to determine the volume of a therapeutic gas actually delivered to the patient by said valve (26) during each inhalation,
a comparator (24) to compare the predetermined volume and the volume of therapeutic gas actually delivered to the patient as determined by said volume monitoring means, said comparator generating a signal based on a difference in such volumes, the control means (24) being arranged to receive said signal from said comparator (24) and to control said timing means (24) to change the duration of time that therapeutic gas is delivered to a patient such that the volume of therapeutic gas actually delivered to a patient is the same as the predetermined volume.

3. A respiratory therapy apparatus as defined in claim 2 wherein said means (38) to establish a predetermined desired volume of therapeutic gas comprises a user input device (38).

4. A respiratory therapy apparatus as defined in claim 3 wherein said conduit (16) has a fixed restrictor (28) and wherein said pressure sensor (30) is located just upstream of said fixed restrictor, and wherein said timing means (24) comprises a central processing unit (CPU) (24) controlling the time said valve (26) is in the open position.

5. A respiratory therapy apparatus as defined in claim 4 wherein said pressure sensor (30) sends a signal to said CPU (24) indicative of the absolute pressure just upstream of said fixed restrictor (28) and wherein said CPU determines the flow through said fixed restrictor based upon the characteristic pressure vs flow through said fixed restrictor for said absolute pressure and wherein said CPU (24) calculates the volume of therapeutic gas through said restrictor (28) to said patient based on said flow determined by said CPU and the time said valve (26) is in said open position.

## Patentansprüche

1. Stickoxid-Atmungstherapievorrichtung zum Verabreichen eines Pulses eines therapeutischen Stickoxidgases an einen Patienten, welche Anordnung umfasst:
Eine unter Druck an eine Therapiegasquelle (10) anschließbare und mit einem Patienten (18) verbindbare Leitung (16),
welche Leitung (16) ein Ventil (26) aufweist, welches den Fluss des therapeutischen Stickoxidgases von einer Quelle zum Patienten steuert, eine Steuerung (24) zum Öffnen und Schließen des Ventils (26) in festgelegten Intervallen, indem sie ein Signal zum Öffnen des Ventils und ein Signal zum Schließen des Ventils sendet,
einen den Druck innerhalb der Leitung (16) stromabwärts des Steuerventils (26) detektierenden Drucksensor (30),
einen Alarm (40, 42),
**dadurch gekennzeichnet,**
**dass** die Vorrichtung ferner umfasst:
Aktivierungsmittel für den Alarm zum Anzeigen einer Fehlfunktion des Ventils (26), wenn (a) das genannte Signal zum Schließen des Ventils (26) von der Steuerung (24) abgesandt wurde und der Drucksensor (30) stromabwärts des Steuerventils keinen Druckabfall um einen bestimmten Betrag detektiert, was darauf hindeutet, dass das Ventil (26) nicht geschlossen wurde, und/oder (b) das genannte Signal zum Öffnen des Ventils (26) von der Steuerung (24) abgesandt wurde und der Drucksensor stromabwärts des Steuerventils keinen Druckanstieg um einen bestimmten Betrag detektiert, was darauf hinweist, dass das Ventil (26) nicht geöffnet wurde.

2. Atmungstherapievorrichtung nach Anspruch 1,
bei welcher die Steuerung eine Zeitsteuerung (24) zum Steuern der Zeitdauer umfasst, in welcher die Verabreichungsmittel das Therapiegas an einen Patienten verabreichen, und bei welcher die Vorrichtung weiterhin umfasst:
Mittel (38) zum Festlegen eines bestimmten gewünschten Volumens eines während jeder Inhalation an einen Patienten zu verabreichenden Therapiegases,
Messelemente (32) zum Erfassen des Beginns einer Inhalation eines Patienten, wobei die Steuerung (24) und das Ventil (26) auf die eine Inhalation eines Patienten erfassenden Messelemente (32) ansprechen, um ein Volumen des Therapiegases während jeder Inhalation des Patienten an diesen zu liefern,
Volumenüberwachungsmittel (24) zum Erfassen des durch das Ventil (26) während jeder Inhalation an den Patienten effektiv verabreichten Volumens eines Therapiegases,
einen Vergleicher (24) zum Vergleichen des Soll-Volumens mit dem tatsächlich an den Patienten abgegebenen Volumen des Therapiegases, wie es von den Volumenüberwachungsmitteln erfasst wird, wobei der Vergleicher ein Signal generiert, welches auf der Differenz dieser Volumina basiert, wobei die Steuerung (24) so ausgestaltet ist, dass sie das genannte Signal vom Vergleicher (24) empfängt und die Zeitsteuerung (24) steuert, um die Zeitdauer, in welcher das Therapiegas an den Patienten verabreicht wird, so zu ändern, dass das Volumen des tatsächlich an den Patienten verabreichten Therapiegases mit dem vorbestimmten Volumen übereinstimmt.

3. Atmungstherapievorrichtung nach Anspruch 2,
bei welcher die Mittel (38) zum Festlegen eines bestimmten gewünschten Volumens an Therapiegas ein Nutzer-Eingabegerät (38) umfasst.

4. Atmungstherapievorrichtung nach Anspruch 3,
bei welcher die Leitung (16) einen starren Begrenzer (28) aufweist, und bei welchem der Drucksensor (30) gleich stromaufwärts des starren Begrenzers angeordnet ist, und bei welchem die Zeitsteuerung (24) eine zentrale Rechnereinheit (central processing unit - CPU) (24) umfasst, welche die Zeit steuert, in der das Ventil (26) sich in der offenen Stellung befindet.

5. Atmungstherapievorrichtung nach Anspruch 4,
bei welcher der Drucksensor (30) ein Signal zur CPU (24) sendet, welches den Absolutdruck gleich oberhalb des starren Begrenzers (28) anzeigt, und bei welcher die CPU den Fluss durch den starren Begrenzer ermittelt, und zwar-basierend auf dem charakteristischen Druck über dem Fluss durch den starren Begrenzer bei dem genannten Absolutdruck, und bei welchem die CPU (24) das Volumen des Therapiegases durch den Begrenzer (28) an den Patienten berechnet, und zwar auf Basis des genannten Flusses, der von der CPU und der Zeit, in welcher das Ventil (26) sich in der offenen Stellung befindet, festgelegt wird.

## Revendications

1. Appareil de thérapie respiratoire à oxyde nitrique pour fournir une impulsion d'oxyde nitrique en tant que gaz thérapeutique à un patient, ledit système comprenant :
un conduit (16) adapté à être connecté à une source du gaz thérapeutique (10) sous pression et à être connecté à un patient (18),
ledit conduit (16) incluant une valve (26) commandant l'écoulement de l'oxyde nitrique, comme gaz thérapeutique depuis une source vers un patient,
des moyens de commande (24) pour ouvrir et fermer ladite valve (26) à des intervalles de temps fixés par envoi d'un signal pour ouvrir ladite valve et d'un signal pour fermer ladite valve,
un capteur de pression (30) adapté à détecter la pression au sein dudit conduit (16) en aval de ladite valve (26) de commande,
une alarme (40,42),
**caractérisé en ce que** l'appareil comprend en outre :
des moyens pour activer ladite alarme indiquant une défaillance de la valve (26) lorsque (a) ledit signal a été envoyé par lesdits moyens de commande (24) pour fermer ladite valve (26) et que ledit capteur de pression (30) ne détecte pas de chute de pression en aval de ladite valve de commande d'une quantité prédéterminée révélatrice du fait que ladite valve (26) ne s'est pas fermée et/ou (b) ledit signal a été envoyé par lesdits moyens de commande (24) pour ouvrir ladite valve (26) et ledit capteur de pression ne détecte pas d'augmentation de la pression en aval de ladite valve de commande d'une quantité prédéterminée révélatrice du fait que la valve (26) ne s'est pas ouverte.

2. Appareil de thérapie respiratoire selon la revendication 1, dans lequel les moyens de commande comprennent des moyens d'horlogerie (24) pour commander la durée pendant laquelle les moyens de fourniture fournissent le gaz thérapeutique à un patient, et dans lequel l'appareil inclut en outre :
des moyens (38) pour établir un volume voulu prédéterminé d'un gaz thérapeutique à délivrer à un patient au cours de chaque inhalation,
des moyens capteurs (32) pour détecter le début d'une inhalation d'un patient, les moyens de commande (24) et la valve (26) étant sensibles auxdits moyens capteurs (32) détectant une inhalation d'un patient pour délivrer un volume du gaz thérapeutique à un patient au cours de chaque inhalation d'un patient,
des moyens de surveillance de volume (24) pour déterminer le volume d'un gaz thérapeutique réellement fourni au patient par ladite valve (26) au cours de chaque inhalation,
un comparateur (24) pour comparer le volume prédéterminé et le volume de gaz thérapeutique réellement fourni au patient, tel que déterminé par ledit moyen de surveillance de volume, ledit comparateur générant un signal reposant sur la différence entre ces volumes, les moyens de commande (24) étant agencés pour recevoir ledit signal depuis ledit comparateur (24) et pour commander lesdits moyens d'horlogerie (24) aux fins de modifier la durée pendant laquelle du gaz thérapeutique est fourni à un patient de telle sorte que le volume de gaz thérapeutique réellement fourni à un patient soit le même que le volume prédéterminé.

3. Appareil de thérapie respiratoire selon la revendication 2, dans lequel lesdits moyens (38) pour établir un volume voulu prédéterminé de gaz thérapeutique comprennent un dispositif (38) permettant à un utilisateur d'entrer des informations.

4. Appareil de thérapie respiratoire selon la revendication 3, dans lequel ledit conduit (16) a un restricteur fixe (28) et dans lequel ledit capteur de pression (30) est situé juste en amont dudit restricteur fixe, et dans lequel lesdits moyens d'horlogerie (24) comprennent une unité centrale de traitement (CPU) (24) commandant le temps pendant lequel la valve (26) est en position ouverte.

5. Appareil de thérapie respiratoire selon la revendication 4, dans lequel ledit capteur de pression (30) envoie un signal à ladite CPU (24) indiquant la pression absolue juste en amont dudit restricteur fixe (28) et dans lequel ladite CPU détermine l'écoulement au travers dudit restricteur fixe sur la base de la pression caractéristique en fonction de l'écoulement au travers dudit restricteur fixe pour ladite pression absolue et dans lequel ladite CPU (24) calcule le volume de gaz thérapeutique passant au travers dudit restricteur (28) vers ledit patient, sur la base dudit écoulement déterminé par ladite CPU (24) et le temps pendant lequel ladite valve (26) est dans ladite position ouverte.
